# EUROPEAN PATENT APPLICATION

(11) **EP 2 248 550 A1**
(43) Date of publication of application: **10.11.2010**
(21) Application number: 09159390.5
(22) Date of filing: 05.05.2009
(51) Int. Cl.: A61M 37/00, A61B 17/32

(54) **Improved surgical instrument**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

A surgical device (10) comprising a grip (11) for holding the surgical device (10), a surgical tool element (12) for use in surgical operations, a drug reservoir (13) for comprising a drug, at least one drug delivery port (16) and an actuator (14) for delivering the drug from the drug reservoir (13), through the at least one drug delivery port (16), into an environment of the surgical tool element (12).

## Description

### FIELD OF THE INVENTION

This invention relates to a surgical device comprising a grip for holding the surgical device and a surgical tool element for use in surgical operations.

### BACKGROUND OF THE INVENTION

Drugs are usually administered in the form of pills. Pills, however, have the disadvantage that they are not very suitable for locally targeting a malfunctioning body part and may cause harmful side effects to body parts not needing any treatment in the first place. For locally targeting specific body parts, surgery and surgical devices may be used. For example, in US patent no. 4,863,457 surgery is used for implanting a device for providing a controlled, localized and sustained release of a therapeutic agent to a patient's eye.

Such implantable devices may however not be suitable for all types of drug and for all positions inside the patient's body. If the implantable device is not fully biodegradable, additional surgery may be needed for removal of waist products.

### OBJECT OF THE INVENTION

It is an object of the invention to provide a better way to administer drugs to a specific local part of a patient's body.

### SUMMARY OF THE INVENTION

According to a first aspect of the invention, this object is achieved by providing a surgical device, comprising a grip for holding the surgical device, a surgical tool element for use in surgical operations, a drug reservoir for comprising a drug and at least one drug delivery port, and an actuator for delivering the drug from the drug reservoir, through the at least one drug delivery port, into an environment of the surgical tool element.

By integrating the drug reservoir and drug supply means (actuator and delivery port) within the surgical device, the need for a separate step of implanting a drug delivery device or supplying the drug is not needed anymore. With the surgical device according to the invention the application of the drugs is made more time efficient. Instead of two separate steps for the surgery and for the application of the drug, only one combined step is needed. The one step procedure further reduces the risk for infections and other complications. The surgical device according to the invention makes it possible to deliver the drugs while the surgeon or doctor is performing the required surgery and exactly at the position where it is needed.

For example, the surgical device may be a scalpel or a clamp. Surgical scalpels are generally available as disposable scalpels, which are thrown away when used, or as reusable handles arranged for receiving disposable cutting blades. A doctor or surgeon uses the scalpel together with clamps and possibly further surgical instruments to be able to expose internal parts of the patient for subsequent inspection, treatment or removal. According to the invention, the drug may be administered while the surgical devices are used. While the surgical device is used, drug release may occur automatically or the surgeon may actively control the drug delivery.

In a preferred embodiment, the actuator is arranged for delivering the drug in discrete doses. The doses may be delivered automatically in accordance with a predetermined time schedule or the user of the surgical device is enabled to control the moment of release of each dose. Alternatively, the drug may be released in response to a certain pressure exerted upon the surgical device or in response to a signal from a sensor included in the surgical device. The user of the device may accurately control the amount of drug to be released during the surgical operation, using configuration means for adjustment of the size of the doses. The configuration means may, e.g., enable the user to adjust the size of the doses mechanically or via a telemetry link.

These and other aspects of the invention are apparent from and will be elucidated with reference to the embodiments described hereinafter.

### BRIEF DESCRIPTION OF THE DRAWINGS

In the drawings:
Fig. 1 shows an example of a known surgical device,
Fig. 2 schematically shows an embodiment of a surgical scalpel according to the invention, and
Fig. 3 schematically shows a further embodiment of a surgical scalpel according to the invention.

### DETAILED DESCRIPTION OF THE INVENTION

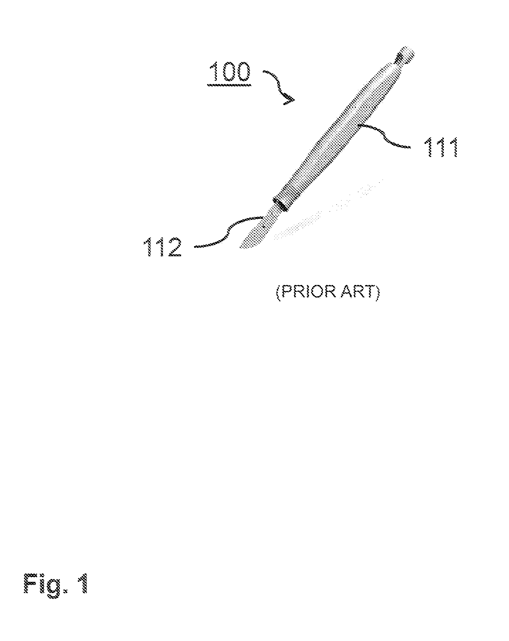

Fig. 1 shows an example of a known surgical device 100. The surgical device in Fig. 1 is a scalpel 100. The scalpel 100 comprises a grip 111 and a cutting blade 112. For hygienic reasons, the cutting blade 112 is used for only one operation or even one incision. The cutting blade 112 may be replaceable, thereby enabling the grip to be used multiple times, while the cutting blade 112 is thrown away and replaced. Alternatively, the scalpel 100 as a whole may be disposable. It is to be noted that the surgical device according to the invention is not necessarily a scalpel 100. It may also be a laparoscopic device, clamp or other type of device used for surgery.

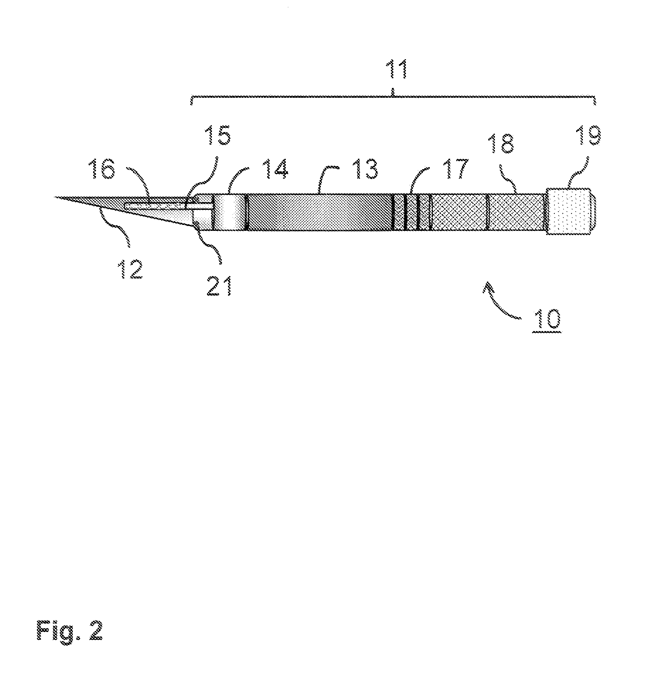

Fig. 2 schematically shows an embodiment of a surgical scalpel 10 according to the invention. Also this scalpel 10 comprises a grip 11 and a cutting blade 12. Again the cutting blade 12 may or may not be replaceable. The grip 11 comprises a drug reservoir 13 for comprising the drug. Depending on the drug release mechanism used, the drugs may be stored in, e.g., liquid form or as a powder. An actuator 14 is provided for taking the drugs from the drug reservoir 13 to one of the delivery ports 16. The delivery ports 16 are preferably arranged at or close to the cutting blade 12, in order to make it possible to release the drug close to where the user is cutting. One delivery port 16 may be sufficient, but more delivery ports 16 may be provided for ensuring a uniform distribution of the drug. A drug channel 15 is provided for directing the drug from the actuator 14 towards the delivery ports 16. The drug reservoir 13 may be replaceable for making it possible to use the scalpel 10 for different types of drug or for installing a full reservoir 13 when the reservoir 13 in use is empty. Alternatively, the drug reservoir 13 may have a filling opening for enabling refilling or the reservoir 13.

The actuator 14 may be implemented as, e.g., a pump mechanism for pumping fluid from the drug reservoir 13 through the drug channel 15 to the delivery ports 14. Alternatively, the actuator 14 may comprise a piston for pushing the drug out of the drug reservoir 13. Many actuator systems for drug release are known from the field of intracorporeal (e.g. swallowable or implantable) drug delivery devices. In principle all these actuator systems may be used for the surgical device 10 according to the invention. The actuator 14 may, e.g., comprise pressurized gas. The actuator system 14 used for this scalpel 100 is controlled by electronics 17 and powered by a battery 18. Alternatively, the actuator 14 may be implemented in a purely mechanical way.

Drug delivery may, e.g., start after the actuator 14 is activated by the user or when a certain pressure is applied to the cutting blade 12. The actuator system 14 may then continuously release drug through the delivery ports 16 into the environment of the cutting blade 12. Alternatively, the actuator 14 may release discrete doses of drugs. The user may be allowed to trigger the release of drugs using a knob or other user control element on the grip 11. A dose configuration knob 19 may be provided for allowing the user to control the size of the doses to be released.

At least one sensor 21 may be provided for, e.g., measuring a temperature or detect a presence or concentration of a certain element in the vicinity of the cutting blade 12. The sensors 21 may be coupled to the electronics 17. The electronics 17 may control the actuator 14 in dependence of the measurements made by the sensor 21. The sensor 21 may also be arranged for sensing a pressure which is exerted upon the cutting blade 12. The drug release may then be initiated when this pressure reaches a critical value. The sensor 21 may function without being coupled to the electronics 17 and in a surgical device 10 without any electronics 17. E.g., the sensor 21 may break or melt under certain circumstances, thereby opening the drug channel 15 and initiating the drug delivery. In such a device, the actuator may, e.g., be a balloon with pressurized gas, which balloon pushes the drug out of the drug reservoir 13 as soon as the drug channel 15 is opened.

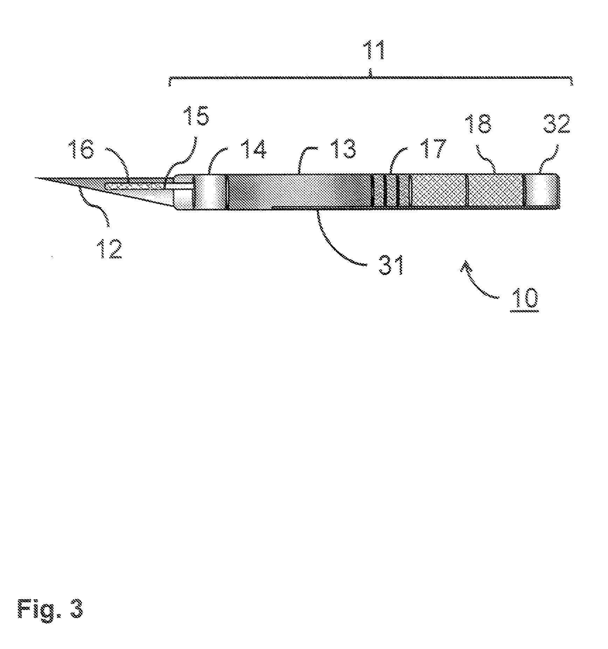

Fig. 3 schematically shows a further embodiment of a surgical scalpel 100 according to the invention. This scalpel 100 comprises a transmitter/receiver 32 for sending data to and receiving data or instructions from an external device. The external device may be a computer system for controlling the drug delivery. An antenna 31 may be comprised in the grip for improving the communication. The transmitter 32 may, e.g., be used for sending sensor data, error codes, drug reservoir 13 filling levels or battery 18 power levels. The receiver 32 may, e.g., be used for receiving dose size configurations and actuator 14 control instructions.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A surgical device (10) comprising:
- a grip (11) for holding the surgical device (10),
- a surgical tool element (12) for use in surgical operations,
- a drug reservoir (13) for comprising a drug, and
- at least one drug delivery port (16), and
- an actuator (14) for delivering the drug from the drug reservoir (13), through the at least one drug delivery port (16), into an environment of the surgical tool element (12).

2. A surgical device (10) as claimed in claim 1, wherein the actuator (14) is arranged for delivering the drug in discrete doses.

3. A surgical device (10) as claimed in claim 2, further comprising configuration means (19, 32) for enabling a user to adjust a size of the discrete doses.

4. A surgical device (10) as claimed in claim 3, wherein the configuration means (19) are arranged for mechanically adjusting the size of the discrete doses.

5. A surgical device (10) as claimed in claim 3, wherein the configuration means (32) are arranged for adjusting the size of the discrete doses via a telemetry link.

6. A surgical device as (10) claimed in claim 1, wherein the actuator (14) comprises an electronic pumping mechanism and wherein the surgical device further discloses electronics (18) for controlling the actuator (14) and a battery (17) for providing electric power to the actuator (14) and the electronics (18).

7. A surgical device (10) as claimed in claim 1, further comprising at least one sensor (21), the device being arranged for delivering the drug in dependence of a signal from the sensor (21).

8. A surgical device (10) as claimed in claim 1, wherein the surgical tool element is a cutting blade (12) for a scalpel (10) and wherein the cutting blade (12) comprises the at least one drug delivery port (16).

9. A surgical device (10) as claimed in claim 1, wherein the surgical tool element is a holder (11) for a replaceable cutting blade (12).

10. A surgical device (10) as claimed in claim 1, wherein the surgical tool element is a laparoscopic tool.

11. A surgical device (10) as claimed in claim 1, wherein the surgical tool element is a clamp.

12. A surgical device (10) as claimed in claim 1, wherein the surgical device is a disposable product.
